# EUROPEAN PATENT APPLICATION

(11) **EP 1 180 349 A1**
(43) Date of publication of application: **20.02.2002**
(21) Application number: 00929792.0
(22) Date of filing: 18.05.2000
(51) Int. Cl.: A61B 18/12

(54) **SNARE WITH RECOVERING IMPLEMENT**

(30) Priority: 19.05.1999 JP 13900299
(71) Applicant: SUMITOMO BAKELITE CO., LTD., Tokyo 140-0002 (JP)
(72) Inventor: Higuma, Masato, Akita Sumitomo Bakelite Co., Ltd., Akita-shi, Akita 011-0951 (JP); Masuda, Haruhiko, Akita Sumitomo Bakelite Co. Ltd, Akita-shi, Akita 011-0951 (JP)
(74) Representative: Savic, Bojan
(86) International application number: JP0003197
(87) International publication number: WO0071042

(57) **Abstract**

Disclosed is a medical device, that is, a snare with a collecting device which can simultaneously resect and collect a diseased portion under a good sight in an endoscopic polyp ablative operation resecting and removing in an endoscopic manner a diseased portion such as a polyp or the like generated within a tissue in a body cavity. In the snare with the collecting device, a collecting device and a collecting device operating fiber are provided at a front end portion of a loop portion in a snare wire of a high-frequency snare.

## Description

### TECHNICAL FIELD

The present invention relates to a treating device for resecting and collecting a tumor and a mucous membrane within a body cavity in an endoscopic manner.

### BACKGROUND ART

In a medical treatment of a benign tumor and a malignant tumor such as a polyp or the like generated in an alimentary canal within the body cavity such as an esophagus, a stomach, a colon and the like, in recent years, an early detection is frequently performed together with a development of an endoscopic examination, a number of cases that the polyp and the tumor are resected and retrieved by an endoscope is rapidly increased. In particular, an endoscopic polypectomy is applied to expanded examples as a minimally invasive medical treatment, and is rapidly spread together with an increase of the case of the polyp and a cancer in an aging society.

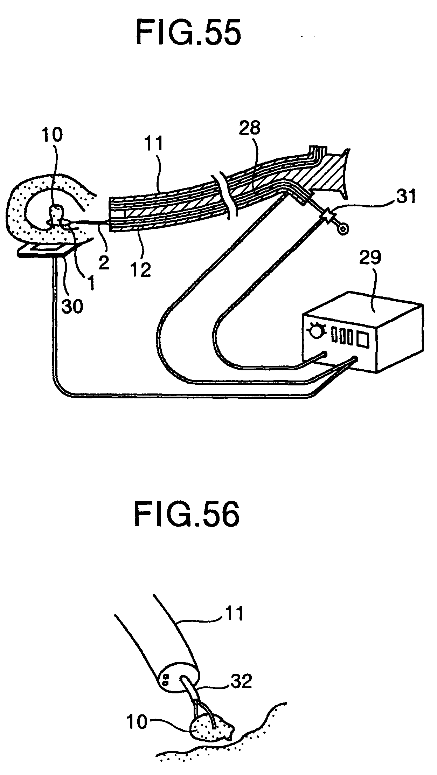

Here, a description will be given of the endoscopic polypectomy. As shown in Fig. 55, an apparatus is constituted by an endoscope 11 for observing an inner portion of a body cavity, a high-frequency snare 28 inserted from a forceps hole 12 of the endoscope so as to strangulate and resect a diseased portion 10, and a high-frequency electro surgical unit 29, and the method is constituted by a step of bringing an earth counter electrode plate 30 into contact with a part of a body such as a foot of a patient from the high-frequency electro surgical unit 29, a step of connecting another to a snare loop 1 of the high-frequency snare 28, a step of applying a high-frequency electric current and a step of cauterising a tissue of the part being contact with the snare loop 1.

A description will be further given of the high-frequency snare. The high-frequency snare is constituted by the snare loop 1 formed in a loop shape at a front end and disposed within a sheath tube 2 corresponding to an outer tube, and a rear end portion 31 for operating so as to take the snare loop 1 in and out the sheath tube 2. Then, the diseased portion 10 is strangulated by hooking the loop portion of the snare wire with the diseased portion 10 such as the polyp or the like and pulling the snare loop 1 into the sheath tube 2, and resecting the diseased portion 10 and stop bleeding thereof are simultaneously executed by applying a high-frequency electric current to the snare loop 1 so as to burn out. The cut-off diseased portion 10 is taken out by inserting a collecting forceps 32 as shown in Fig. 56 from the forceps hole 12 in the endoscope, gripping the diseased portion 10 and drawing out from outside the body together with the endoscope. Thereafter, the diseased portion 10 is judged in accordance with a pathologically test whether it is malignant or benign, and there can be obtained an information relating to a degree of development of a cancer focus and the like. The results mentioned above become a most important information in view of determining the future medical treatment policy.

Here, in the case of gripping the diseased portion 10 after being resected by the collecting forceps 32, in the endoscope 11 provided with one channel forceps hole 12, since it is necessary to once take out the high-frequency snare 28 used for resecting from the forceps hole and replacing by the collecting forceps 32, it takes a lot of trouble for an operator and it also takes a lot of time. In some cases, the resected diseased portion 10 is moved due to a breathing movement or the like during a replacement from the high-frequency snare 28 to the collecting forceps 32, and there is a case of losing sight of the diseased portion 10. In particular, in the colon, since it is hard to fine the resected diseased portion 10 due to an unevenness of the alimentary canal or the like and an operability of the endoscope 11 is bad in comparison with an upper alimentary canal, there is a case that it is impossible to retrieve, whereby it becomes impossible to execute the pathological test. Further, when the disease portion 10 is large, there is a case that it is impossible to take out it by one time and it is separately retrieved by two times, or a plurality of polyps exist. Accordingly, since the resecting operation and the collecting operation are repeated, a load applied to the operator and a time for medical treatment are increased, and a load applied to the patient is increased.

On the contrary, if the endoscope 11 has two channels of forceps holes 12, the endoscope 11 can be used by inserting the high-frequency snare 28 to one and inserting the collecting forceps 32 to another, however, not only an outer diameter thereof is increased and an operability is deteriorated in comparison with the endoscope 11 having one channel, but also a pain of the patient at a time of inserting is increased and a cost becomes expensive, so that this type of endoscope 11 is not so popular in general hospitals.

On the contrary, in order to achieve the problems mentioned above, there has been conventionally considered a capturing method by a net. This is a two-lumen tube in which the sheath tube 2 is constituted by two holes, and a collecting device with the high-frequency snare 28 in one and a collection net in another is received within the sheath tube 2 in accordance with an operation of respective end portions. In the case of the collecting device mentioned above, at first, the collection net in a state of being received within the sheath tube 2, the snare loop resects the polyp, and thereafter protrudes the collection net from a front end of the sheath tube 2 so as to collect the polyp, however, the diseased portion 10 frequently jumps due to a rebound caused by resecting the polyp and goes out of sight of the endoscope 11, so that there is a problem that a lot of time is required for searching for the collecting.

Then, for the purpose of collecting at the same time of resecting by the snare loop 1, there has been invented a structure in which a collection net 33 employing an insulative thread is directly mounted to the snare loop 1 portion, as shown in Fig. 5 (U.S.P. Nos. 5,190,542, 5,201,740, 5,336,227 and 5,486,182, and PCT/US92/09299), however, since in a fixing portion of the collection net 33 to the snare loop 1, there is generated a portion in which the conductive snare loop 1 is not directly in contact with the polyp, there is a case that a cauterization time is extended so as to generate a reduction of the resecting capacity, and the collection net 33 itself melts down due to a heat of cauterization so as to be broken, and further, the collection net 33 blocks the operator's view at a time of strangulating the polyp. Further, there is a problem that the collection net 33 is caught on the sheath tube at a time of receiving the snare loop 1, whereby it is hard to recognize a degree of strangulation of the disease on the basis of a response of a handling operation.

Further, there have been made an invention (JP-A-6-217985) that a wire is connected to a front end of the snare loop 1 and an angle and a direction of the snare loop 1 are adjusted by drawing the wire, thereby making it easy to approach the diseased portion 10, and an invention (JP-B2-2997484) that a receiving portion is provided at a front end of a basket-like gripping portion and a plurality of foreign materials can be taken in by pressing once gripped and taken-in foreign materials to the receiving portion without taking out the endoscope, however, these are different from the object mentioned above, and the problems can not be solved.

Further, there has been made an invention (JP-A-9-313499) that a retrieving means is provided at a front end, whereby the diseased portion resected by the high-frequency is received in the retrieving means at the front end, and one or a plurality of polyps can be resected without taking out the endoscope, however, since this structure does not grip the diseased portion after resecting the diseased portion by the snare loop 1, the diseased portion 10 frequently jumps due to the rebound caused by resecting the polyps mentioned above and goes out of the sight of the endoscope 11, so that it has not effect for solving the problem that a lot of time is required for searching for the collecting.

There has been made an invention (JP-A-11-503631) of a type that a collecting means for collecting the diseased portion 10 at the same time of resecting the diseased portion 10 is achieved by fixing the diseased portion 10 by the forceps taken out from the sheath tube 2, formed in a rod shape and having a projection at a front end at a time of strangulating the diseased portion 10 by the snare loop 1 so as to collect the diseased portion 10 at the same time of resecting. However, since the invention is of a type of hooking to the diseased portion 10 only by some projections, there is a great possibility that the diseased portion 10 spills out due to the rebound at a time of resecting, and it is hard to securely resect and simultaneously collect. Further, in accordance with the invention, there has been invented a structure that a clip-like ring is provided at the front end of the snare loop 1 so as to press the diseased portion 10 in the ring after resecting, however, this structure has a high possibility that the diseased portion 10 spills out due to the rebound at a time of resecting the diseased portion 10 in the same manner, and it is hard to securely collect the diseased portion 10.

### DISCLOSURE OF THE INVENTION

The present invention is made as a result of considering the problems mentioned above as problems to be solved, and an object of the present invention is to provide a high-frequency snare for simply and securely resecting and retrieving a diseased portion such as a polyps or the like.

In accordance with an aspect of the present invention, there is provided a snare with a collecting device comprising:
a snare wire made of a conductive wire and forming a loop portion at a front end;
the snare wire being connected to a handle portion at a rear end through a sheath tube;
the snare loop being moved forward and backward by operating the handle portion; and
a high-frequency snare received in the sheath tube and resecting a diseased portion such as a polyp or the like generated in a tissue within a body cavity in an endoscopic manner,
wherein the collecting device is provided at a leading end of the loop portion of the snare wire, and one or a plurality of collecting device operating fibers are connected to the collecting device.

Further, in accordance with the other aspects of the present invention, the structure may be made such that the collecting device is constituted by a net, or a sheet or a combination thereof, that the collecting device has a turning point and has a function of bending at the turning point, that the collecting device has a turning point and has a function that the collecting device bends in a vertical direction at the turning point, that the collecting device has a turning point and has a function that the collecting device bends in a vertical direction and a longitudinal direction at the turning point, that the collecting device operating fiber is red, blue, green, white or yellow, that the collecting device operating fiber is red, blue, green, white or yellow and two colors or a plurality of colors among them form a stripe-like arranged color, and that a collecting device operating rod provided in the handle portion is connected to a snare operating rod provided in the handle portion in the same manner and a relative position between both of the operating rods is capable of being changed within a fixed distance.

### BRIEF DESCRIPTION OF THE DRAWINGS

Fig. 1 is a schematic view of a snare portion of a snare with a collecting device in accordance with the present invention, which shows the collecting device formed in a hexagonal shape by using a net and provided with two turning points;
Figs. 2 to 8 are schematic views showing an operation from resecting a diseased portion to collecting the diseased portion in accordance with the present invention;
Fig. 9 is a schematic view of a snare portion of a snare with a collecting device in accordance with the present invention, which shows the collecting device formed in a rhombic shape by using a net and provided with two turning points;
Fig. 10 is a schematic view of a snare portion of a snare with a collecting device in accordance with the present invention, which shows the collecting device formed in a circular arc shape by using a net and provided with two turning points;
Fig. 11 is a schematic view of a snare portion of a snare with a collecting device in accordance with the present invention, which shows the collecting device formed in a hexagonal shape by using a sheet and provided with two turning points;
Fig. 12 is a schematic view of a snare portion of a snare with a collecting device in accordance with the present invention, which shows the collecting device formed in a rhombic shape by using a sheet and provided with two turning points;
Fig. 13 is a schematic view of a snare portion of a snare with a collecting device in accordance with the present invention, which shows the collecting device formed in a circular arc shape by using a sheet and provided with two turning points;
Fig. 14 is a schematic view of a snare portion of a snare with a collecting device in accordance with the present invention, which shows the collecting device formed in an octagonal shape by using a net and provided with two turning points and one front end turning point;
Figs. 15 to 21 are schematic views showing an operation from resecting a diseased portion to collecting the diseased portion in accordance with the present invention;
Fig. 22 is a schematic view of a snare portion of a snare with a collecting device in accordance with the present invention, which shows the collecting device formed in a rhombic shape by using a net and provided with two turning points and one front end turning point;
Fig. 23 is a schematic view of a snare portion of a snare with a collecting device in accordance with the present invention, which shows the collecting device formed in a circular arc shape by using a net and provided with two turning points and one front end turning point;
Fig. 24 is a schematic view of a snare portion of a snare with a collecting device in accordance with the present invention, which shows the collecting device formed in an octagonal shape by using a sheet and provided with two turning points and one front end turning point;
Fig. 25 is a schematic view of a snare portion of a snare with a collecting device in accordance with the present invention, which shows the collecting device formed in a rhombic shape by using a sheet and provided with two turning points and one front end turning point;
Fig. 26 is a schematic view of a snare portion of a snare with a collecting device in accordance with the present invention, which shows the collecting device formed in a circular arc shape by using a sheet and provided with two turning points and one front end turning point;
Fig. 27 is a schematic view of a snare portion of a snare with a collecting device in accordance with the present invention, which shows the collecting device formed in a rhombic shape by using a net and provided with two turning points and one front end turning point;
Fig. 28 is a schematic view of a snare portion of a snare with a collecting device in accordance with the present invention, which shows the collecting device formed in a pentagonal shape by using a net and provided with one front end turning point;
Figs. 29 to 35 are schematic views showing an operation from resecting a diseased portion to collecting the diseased portion in accordance with the present invention;
Fig. 36 is a schematic view of a snare portion of a snare with a collecting device in accordance with the present invention, which shows the collecting device formed in a triangular shape by using a net and provided with one front end turning point;
Fig. 37 is a schematic view of a snare portion of a snare with a collecting device in accordance with the present invention, which shows the collecting device formed in a circular arc shape by using a net and provided with one front end turning point;
Fig. 38 is a schematic view of a snare portion of a snare with a collecting device in accordance with the present invention, which shows the collecting device formed in a pentagonal shape by using a sheet and provided with one front end turning point;
Fig. 39 is a schematic view of a snare portion of a snare with a collecting device in accordance with the present invention, which shows the collecting device formed in a triangular shape by using a sheet and provided with one front end turning point;
Fig. 40 is a schematic view of a snare portion of a snare with a collecting device in accordance with the present invention, which shows the collecting device formed in a circular arc shape by using a sheet and provided with one front end turning point;
Fig. 41 is a top elevational view schematically showing a whole of a snare with a collecting device including a handle portion in accordance with the present invention, in which the snare is constituted by a handle main body and a slide portion and is provided with a collecting device operating lever;
Fig. 42 is a side elevational view schematically showing a whole of the snare with the collecting device including the handle portion in accordance with the present invention, in which the snare is constituted by the handle main body and the slide portion and is provided with the collecting device operating lever;
Figs. 43 to 46 are schematic views showing an operation of the handle portion in accordance with the present invention;
Fig. 47 is a top elevational view schematically showing a whole of a snare with a collecting device including a handle portion in accordance with the present invention, in which the snare is constituted by a handle main body and a slide portion;
Fig. 48 is a side elevational view schematically showing a whole of the snare with the collecting device including the handle portion in accordance with the present invention, in which the snare is constituted by the handle main body and the slide portion;
Figs. 49 to 52 are schematic views showing an operation of the handle portion in accordance with the present invention;
Fig. 53 is a schematic view of a whole of a snare with a collecting device including a handle portion in accordance with the present invention, in which the snare is constituted by a snare portion operating handle and a collecting portion operating handle;
Fig. 54 is a schematic view showing a high-frequency snare in accordance with a conventional art, showing a structure in which a collection net using an insulative thread is directly mounted to a loop portion of a snare wire;
Fig. 55 is a view showing a used state of a high-frequency resecting tool; and
Fig. 56 is a view showing a collecting forceps.

### BEST MODE FOR CARRYING OUT THE INVENTION

A high-frequency resecting tool for resecting a diseased portion in accordance with the present invention basically has the same structure as that of the conventional high-frequency snare, and has an aspect in which an assisting tool for collecting is additionally provided. The high-frequency snare is structured such that a snare loop 1 is provided so as to form a loop portion at a front end constituted by a conductive wire, a wire connected to the snare loop 1 is connected to a handle portion mentioned below through a sheath tube 2 and the snare loop 1 is moved forward and backward by operating the handle portion, thereby making it possible to optionally take in and out from the sheath tube 2.

### "Embodiment 1 of snare portion"

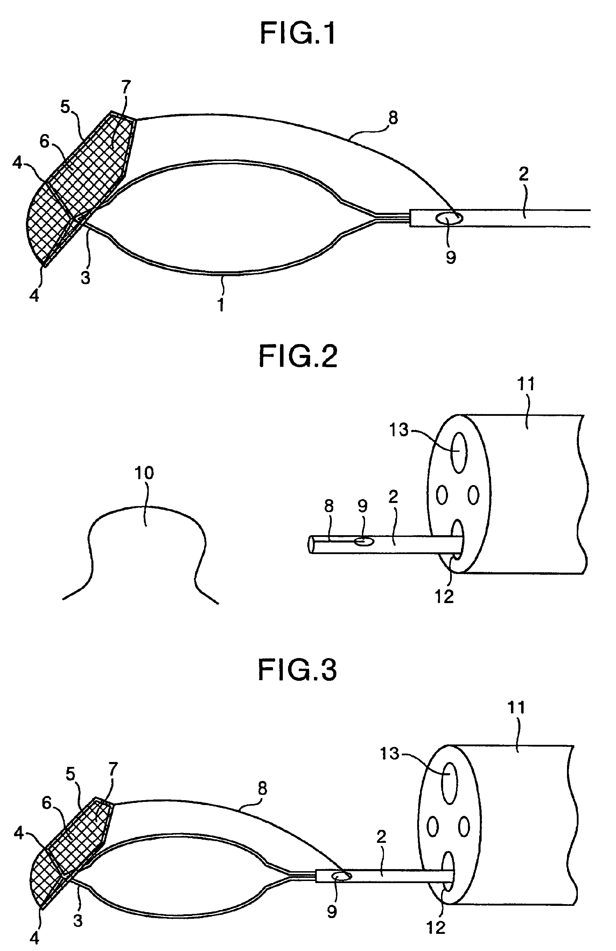

Fig. 1 shows an embodiment in accordance with the present invention in a state of completely taking out the snare loop 1 from the sheath tube 2. As shown in Fig. 1, at first, a collecting device 7 constituted by a hexagonal net frame 5 having two forward turning points 4 and a net 6 surrounded by the net frame 5 is provided in a snare front end portion 3 corresponding to a front end portion of the snare loop 1, and a collecting device operating fiber 8 is connected to an upper portion of the collecting device 7. Another end of the collecting device operating fiber 8 opposite to the end connected to the collecting device 7 is inserted to an inner hole of the sheath tube 2 from an opening portion 9 open on a surface of the sheath tube 2 and is indirectly connected to a handle portion.

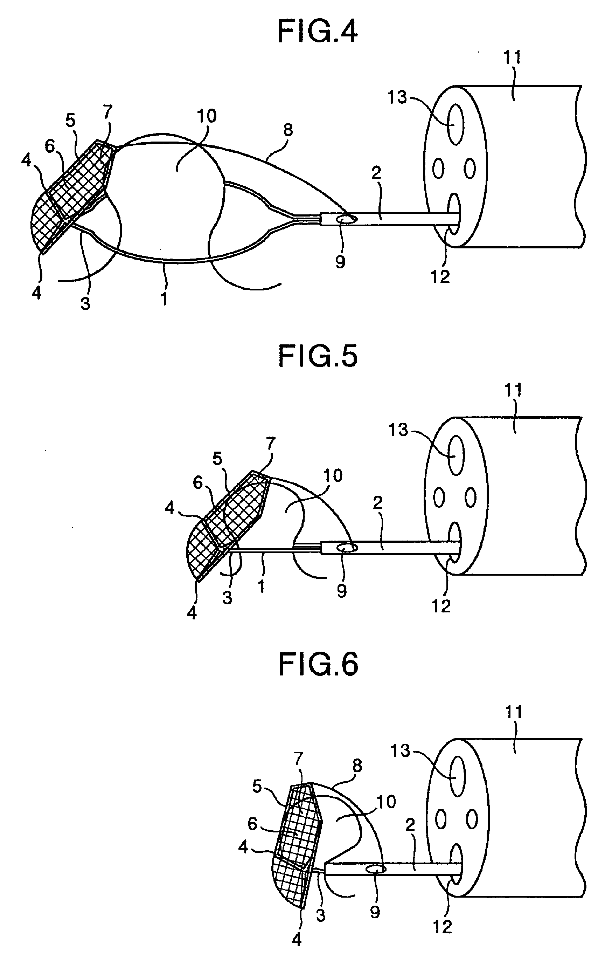

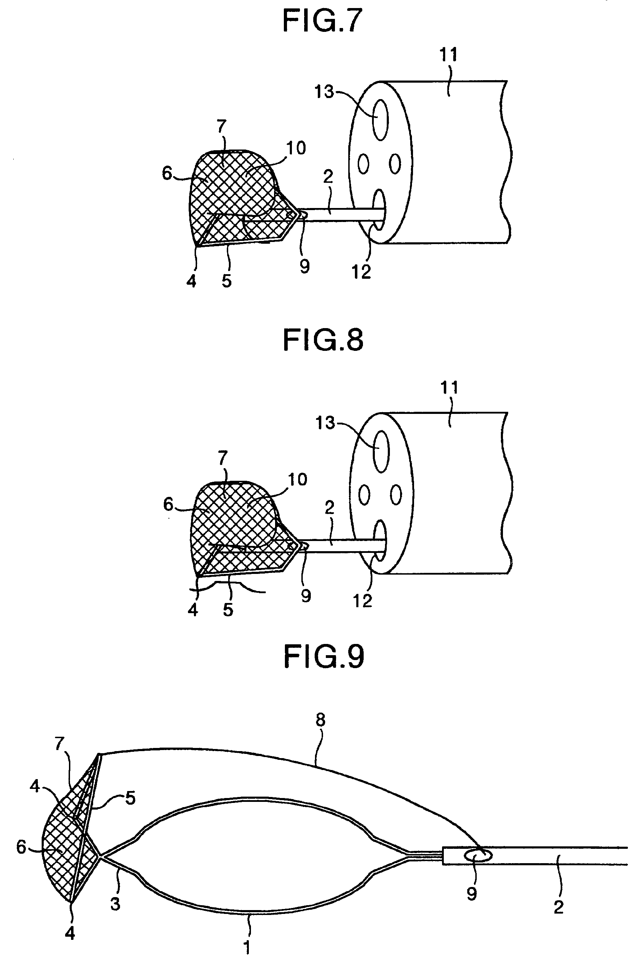

A description will be given of an operation of the snare with the collecting device in accordance with the present invention with reference to Figs. 2 to 8. At first, a diseased portion 10 is found on the basis of an endoscopic observation, and the snare with the collecting device is pressed out from a forceps hole 12 of an endoscope 11 as shown in Fig. 2. At this time, all of a snare portion including the snare loop 1 and the collecting device 7 is received within the sheath tube 2. Next, the snare portion is taken out from the sheath tube 2 in accordance with an operation by the handle portion as shown in Fig. 3, and the diseased portion 10 is taken within the snare loop 1 as shown in Fig. 4. At this time, since there is no visual obstacle existing between an endoscope lens 13 and the diseased portion 10 taken within the snare loop 1, it is possible to operate under the same good visual field as that at a time of singly using the high-frequency snare. Sequentially, the snare portion is again received within the sheath tube 2 in accordance with an operation by the handle portion as shown in Fig. 6 from Fig. 5, and the diseased portion 10 is strangulated as shown in Fig. 6. At this time, the collecting device 7 is in contact with the diseased portion 10 as shown in Fig. 6. Next, when the collecting device operating fiber 8 is drawn backward in accordance with the operation of the handle portion and a portion of the collecting device 7 to which the collecting device operating fiber 8 is connected is moved close to the opening portion 9, the collecting device 7 bends-at the turning point 4 as shown in Fig. 7 and the collecting device 7 covers the strangulated diseased portion 10 from the above so as to fix the diseased portion 10. When fixing the diseased portion 10 is finished, a high-frequency electric current is applied to the snare loop 1 as shown in Fig. 8 and the strangulated portion of the diseased portion 10 is cauterized. At this time, since a portion which is strangulated in and in contact with the diseased portion 10 is only a snare wire, and no insulating material exists between the both, a cauterizing capacity of the high-frequency snare itself is not restricted. Since the diseased portion 10 is fixed by the collecting device 7 after being cauterized, the endoscope 11 is removed from the patient under the state, whereby the diseased portion 10 is taken out of the body.

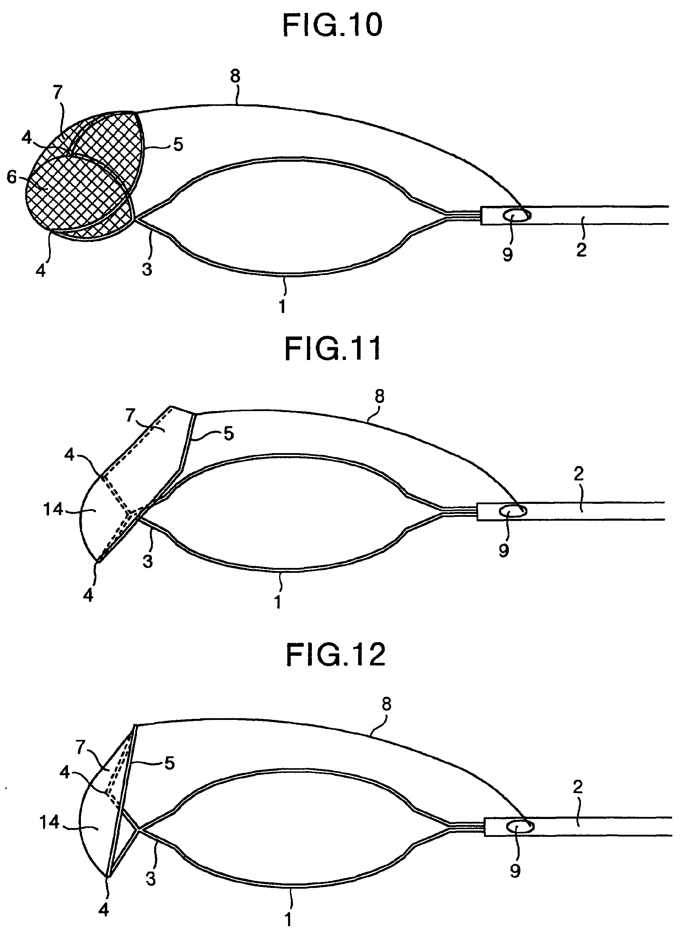

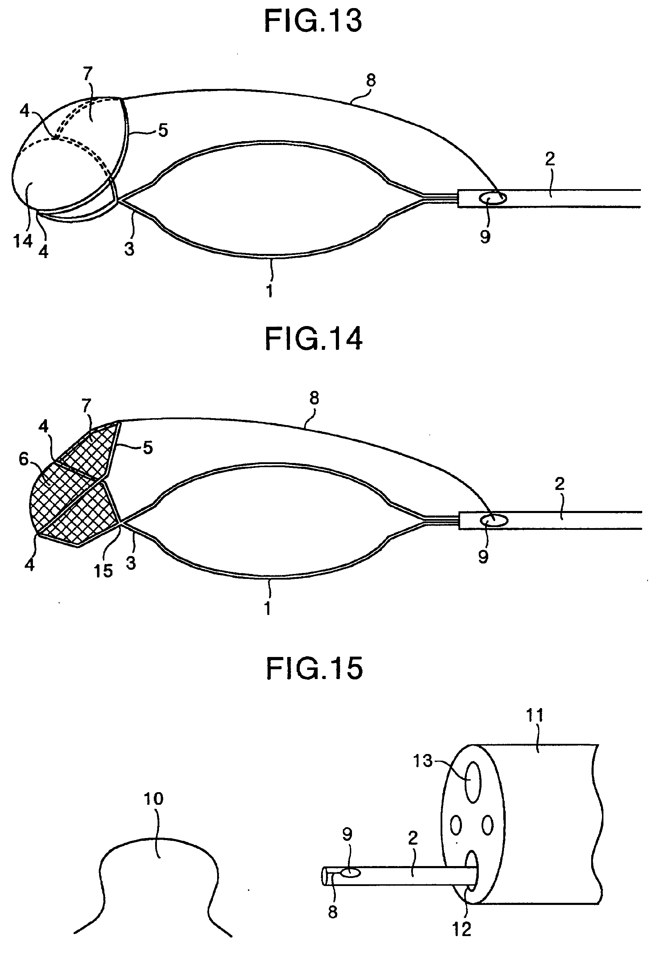

A shape of the collecting device 7 is not limited, in accordance with the present embodiment, it is sufficient to consider that the turning points 4 are provided so that the collecting device 7 forms a bending shape covering the diseased portion 10 from the above at a time of fixing the diseased portion 10 as mentioned above and this can collect an expected size of the diseased portion 10. As embodiments of the shape of the collecting device 7, the following structures are listed up. Fig. 9 shows a structure in which the collecting device 7 is formed in a rhombic shape, and Fig. 10 shows a structure in which the collecting device 7 is formed in a circular arc shape. Further, in place of the net 6 mentioned above, a thin sheet 14 may be employed. Fig. 11 shows a structure in which the collecting device 7 is formed in a hexagonal shape, Fig. 12 shows a structure in which the collecting device 7 is formed in a rhombic shape, and Fig. 13 shows a structure in which the collecting device 7 is formed in a circular arc shape.

The collecting device 7 requires a size capable of covering the expected diseased portion 10 so as to fix, that is, if the expected size of the diseased portion 10 is 5 to 15 mm, it is recommended that a width of the collecting device 7 is 10 to 20 mm and a length thereof is about 12 to 25 mm, however, this is not limited.

Further, it is preferable that a position of the opening portion 9 is set so that a distance between the binding point 4 and the opening portion 9 becomes substantially equal to a distance between the binding point 4 and the portion of the collecting device 7 connected to the collecting device operating fiber 8 at a time of fixing the diseased portion 10 mentioned above, and it is preferable that the position of the opening portion 9 is set at 2 to 15 mm apart from the leading end and further desirably set at 2 to 10 mm.

Due to the opening portion 9, since an intended motion of the collecting device 7 as shown in Fig. 7 can be achieved, and a distance of the collecting device operating fiber 8 from the leading end portion of the sheath tube 2 generating a high heat at a time of cauterizing shown in Fig. 8 can be secured so that the collecting device operating fiber 8 is not broken due to the heat, there can be obtained an effect that no specific heat resisting property is not required for the collecting device operating fiber 8.

### "Embodiment 2 of snare portion"

Fig. 14 shows another embodiment in accordance with the present invention in a state of completely taking out the snare loop 1 from the sheath tube 2, in which an operation of the collecting device 7 is differentiated from the embodiment shown in Fig. 1. As shown in Fig. 14, at first, a collecting device 7 constituted by an octagonal net frame 5 having two turning points 4 and one front end turning point 15 and a net 6 surrounded by the net frame 5 is provided in a snare front end portion 3 corresponding to a front end portion of the snare loop 1, and a collecting device operating fiber 8 is connected to an upper portion of the collecting device 7. Another end of the collecting device operating fiber 8 opposite to the end connected to the collecting device 7 is inserted to an inner hole of the sheath tube 2 from an opening portion 9 open on a surface of the sheath tube 2 and is indirectly connected to a handle portion.

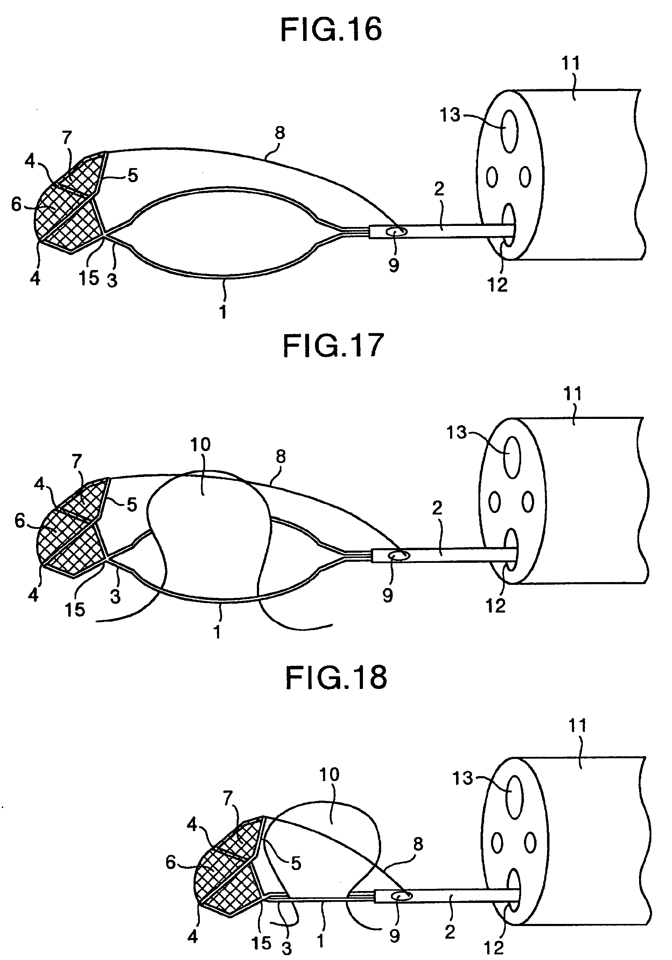

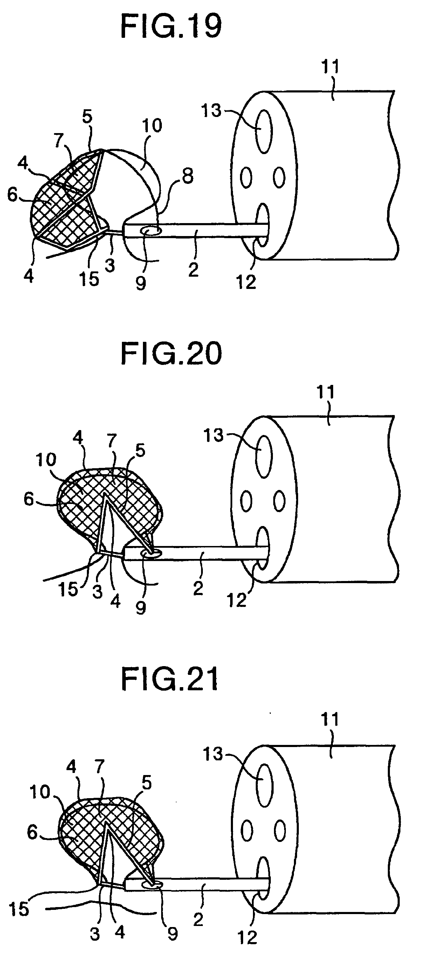

A description will be given of an operation of the snare with the collecting device in accordance with the present invention with reference to Figs. 15 to 21. At first, a diseased portion 10 is found on the basis of an endoscopic observation, and the snare with the collecting device is pressed out from a forceps hole 12 of an endoscope 11 as shown in Fig. 15. At this time, all of a snare portion including the snare loop 1 and the collecting device 7 is received within the sheath tube 2. Next, the snare portion is taken out from the sheath tube 2 in accordance with an operation by the handle portion as shown in Fig. 16, and the diseased portion 10 is taken within the snare loop 1 as shown in Fig. 17. At this time, since there is no visual obstacle existing between an endoscope lens 13 and the diseased portion 10 taken within the snare loop 1, it is possible to operate under the same good visual field as that at a time of singly using the high-frequency snare. Sequentially, the snare portion is again received within the sheath tube 2 in accordance with an operation by the handle portion as shown in Fig. 19 from Fig. 18, and the diseased portion 10 is strangulated as shown in Fig. 19. At this time, the collecting device 7 is in contact with the diseased portion 10 as shown in Fig. 19. Next, when the collecting device operating fiber 8 is drawn backward in accordance with the operation of the handle portion and a portion of the collecting device 7 to which the collecting device operating fiber 8 is connected is moved close to the opening portion 9, the collecting device 7 bends at the turning point 4 and the front end turning point 15 as shown in Fig. 20 and the collecting device 7 covers the strangulated diseased portion 10 from the above and fixes the diseased portion 10 so as to grip in a longitudinal direction. When fixing the diseased portion 10 is finished, a high-frequency electric current is applied to the snare loop 1 as shown in Fig. 21 and the strangulated portion of the diseased portion 10 is cauterized. At this time, since a portion which is strangulated in and in contact with the diseased portion 10 is only a snare wire, and no insulating material exists between the both, a cauterizing capacity of the high-frequency snare itself is not restricted. Since the diseased portion 10 is fixed by the collecting device 7 after being cauterized, the endoscope 11 is removed from the patient under the state, whereby the diseased portion 10 is taken out of the body.

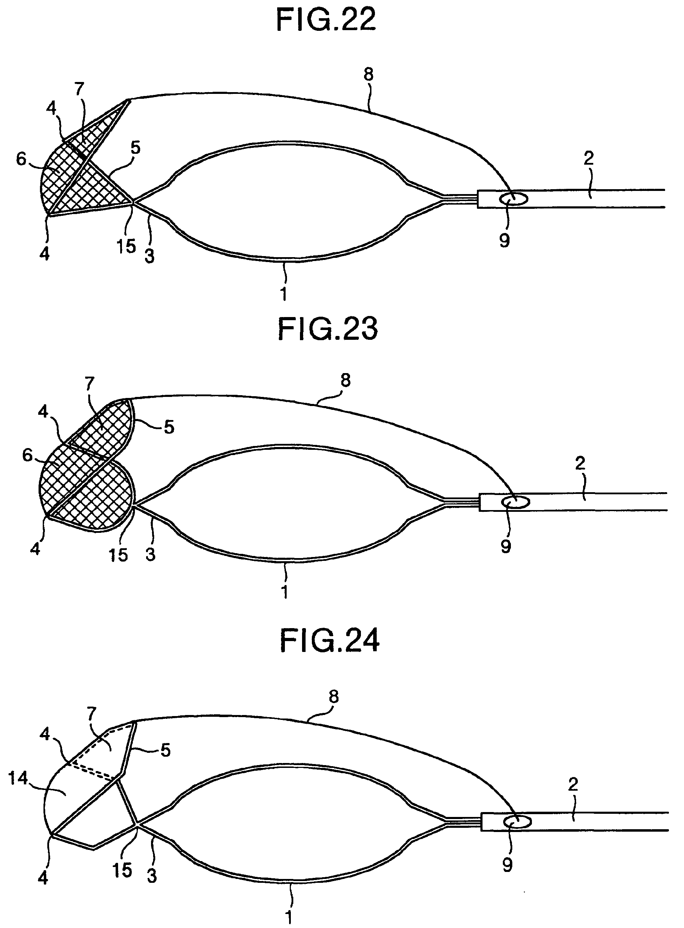

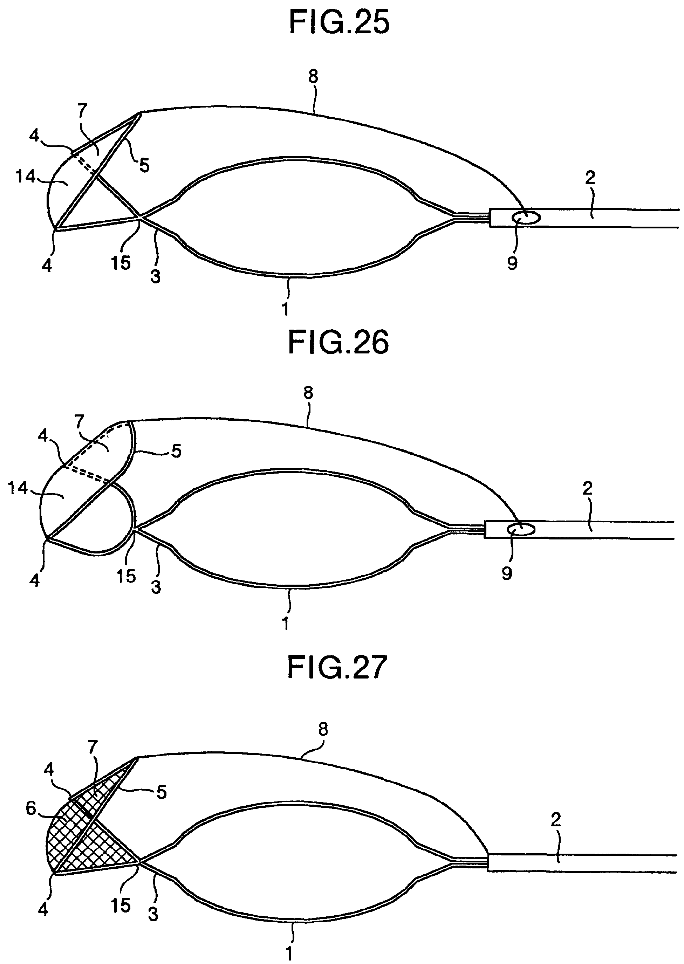

A shape of the collecting device 7 is not limited, in accordance with the present embodiment, it is sufficient to consider that the turning points 4 and the front end turning point 15 are provided so that the collecting device 7 forms a bending shape covering the diseased portion 10 from the above at a time of fixing the diseased portion 10 as mentioned above and gripping the diseased portion 10 from the longitudinal direction and this can collect an expected size of the diseased portion 10. As embodiments of the shape of the collecting device 7, the following structures are listed up. Fig. 22 shows a structure in which the collecting device 7 is formed in a rhombic shape, and Fig. 23 shows a structure in which the collecting device 7 is formed in a circular arc shape. Further, in place of the net 6 mentioned above, a thin sheet 14 may be employed. Fig. 24 shows a structure in which the collecting device 7 is formed in an octagonal shape, Fig. 25 shows a structure in which the collecting device 7 is formed in a rhombic shape, and Fig. 26 shows a structure in which the collecting device 7 is formed in a circular arc shape.

The collecting device 7 requires a size capable of covering the expected diseased portion 10 so as to fix, that is, if the expected size of the diseased portion 10 is 5 to 15 mm, it is recommended that a width of the collecting device 7 is 10 to 20 mm and a length thereof is about 12 to 25 mm, however, this is not limited.

Further, it is preferable that a position of the opening portion 9 is set so that a distance between the front end turning point 15 and the opening portion 9 becomes about 1 to 5 mm at a time of fixing the diseased portion 10 as shown in Fig. 20, and it is preferable that the position of the opening portion 9 is set at 1 to 4 mm apart from the leading.

Due to the opening portion 9, since an intended motion of the collecting device 7 as shown in Fig. 20 can be achieved, and a distance of the collecting device operating fiber 8 from the leading end portion of the sheath tube 2 generating a high heat at a time of cauterizing shown in Fig. 21 can be secured so that the collecting device operating fiber 8 is not broken due to the heat, there can be obtained an effect that no specific heat resisting property is not required for the collecting device operating fiber 8.

Further, the structure may be made such that the opening portion 9 is cancelled as shown in Fig. 27 and the collecting device operating fiber 8 goes out of the inner hole of the sheath tube 2 from a portion close to a portion through which the snare loop 1 at the leading end of the sheath tube 2 passes. However, in this case, since the front end of the sheath tube 2 generates a high heat, a heat resisting property is required in the collecting device operating fiber 8 for the purpose of preventing the collecting device operating fiber 8 from being damaged due to the heat.

### "Embodiment 3 of snare portion"

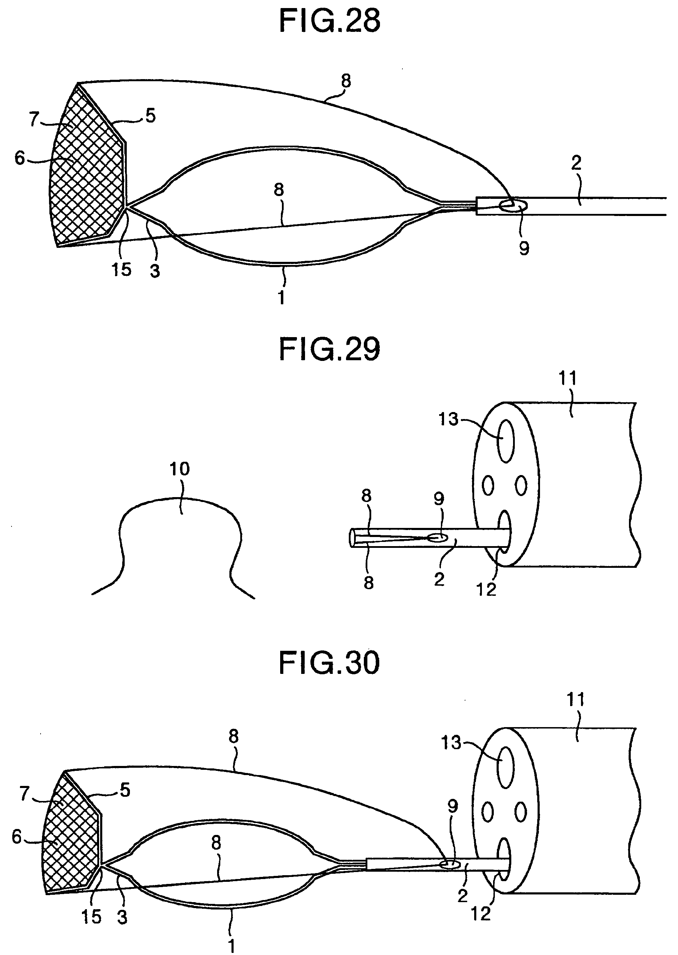

Fig. 28 shows the other embodiment in accordance with the present invention in a state of completely taking out the snare loop 1 from the sheath tube 2, in which an operation of the collecting device 7 is differentiated from the embodiment shown in Figs. 1 and 14. As shown in Fig. 28, at first, a collecting device 7 having one front end turning point 15 and constituted by two net frames 5 and a net 6 provided between two net frames 5 so as to be formed in a pentagonal shape as a whole is provided in a snare front end portion 3 corresponding to a front end portion of the snare loop 1, and a collecting device operating fiber 8 is connected to an upper portion of each of two net frames 5. Another end of the collecting device operating fiber 8 opposite to the end connected to the collecting device 7 is inserted to an inner hole of the sheath tube 2 from an opening portion 9 open on a surface of the sheath tube 2 and is indirectly connected to a handle portion.

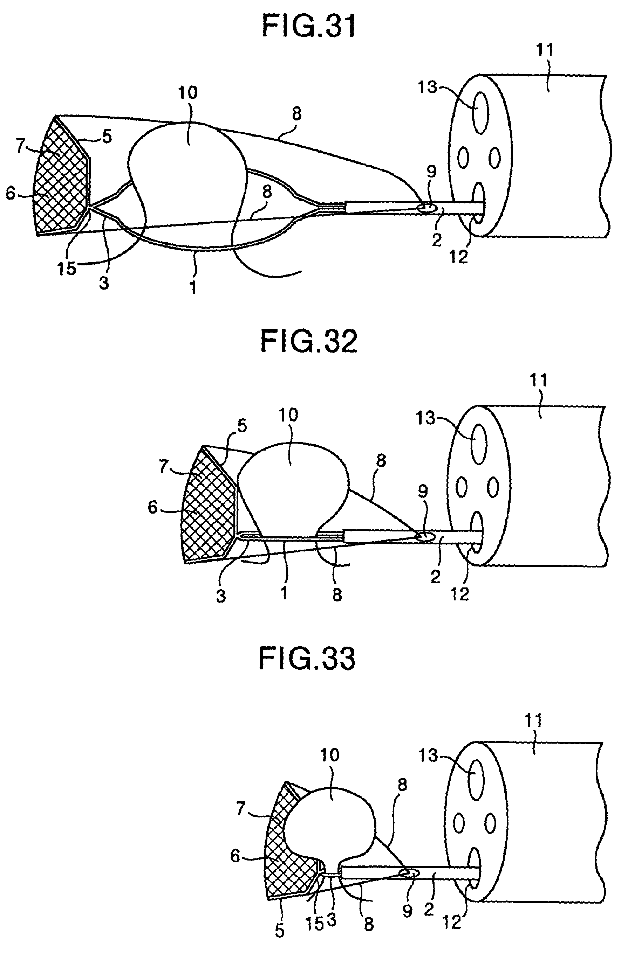

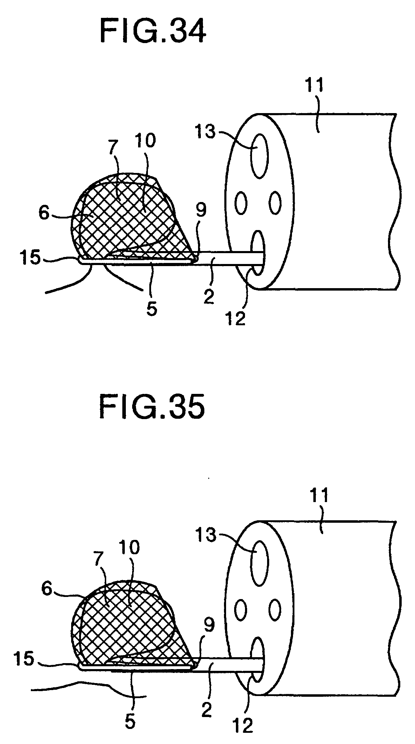

A description will be given of an operation of the snare with the collecting device in accordance with the present invention with reference to Figs. 29 to 35. At first, a diseased portion 10 is found on the basis of an endoscopic observation, and the snare with the collecting device is pressed out from a forceps hole 12 of an endoscope 11 as shown in Fig. 29. At this time, all of a snare portion including the snare loop 1 and the collecting device 7 is received within the sheath tube 2. Next, the snare portion is taken out from the sheath tube 2 in accordance with an operation by the handle portion as shown in Fig. 30, and the diseased portion 10 is taken within the snare loop 1 as shown in Fig. 31. At this time, since there is no visual obstacle existing between an endoscope lens 13 and the diseased portion 10 taken within the snare loop 1, it is possible to operate under the same good visual field as that at a time of singly using the high-frequency snare. Sequentially, the snare portion is again received within the sheath tube 2 in accordance with an operation by the handle portion as shown in Fig. 33 from Fig. 32, and the diseased portion 10 is strangulated as shown in Fig. 33. At this time, the collecting device 7 is in contact with the diseased portion 10 as shown in Fig. 33. Next, when the collecting device operating fiber 8 is drawn backward in accordance with the operation of the handle portion and a portion of two collecting devices 7 to which the collecting device operating fiber 8 is connected is moved close to the opening portion 9, the collecting device 7 bends at the front end turning point 15 as shown in Fig. 34 and the collecting device 7 fixes the diseased portion 10 so as to cover the strangulated diseased portion 10 from the above. When fixing the diseased portion 10 is finished, a high-frequency electric current is applied to the snare loop 1 as shown in Fig. 35 and the strangulated portion of the diseased portion 10 is cauterized. At this time, since a portion which is strangulated in and in contact with the diseased portion 10 is only a snare wire, and no insulating material exists between the both, a cauterizing capacity of the high-frequency snare itself is not restricted. Since the diseased portion 10 is fixed by the collecting device 7 after being cauterized, the endoscope 11 is removed from the patient under the state, whereby the diseased portion 10 is taken out of the body.

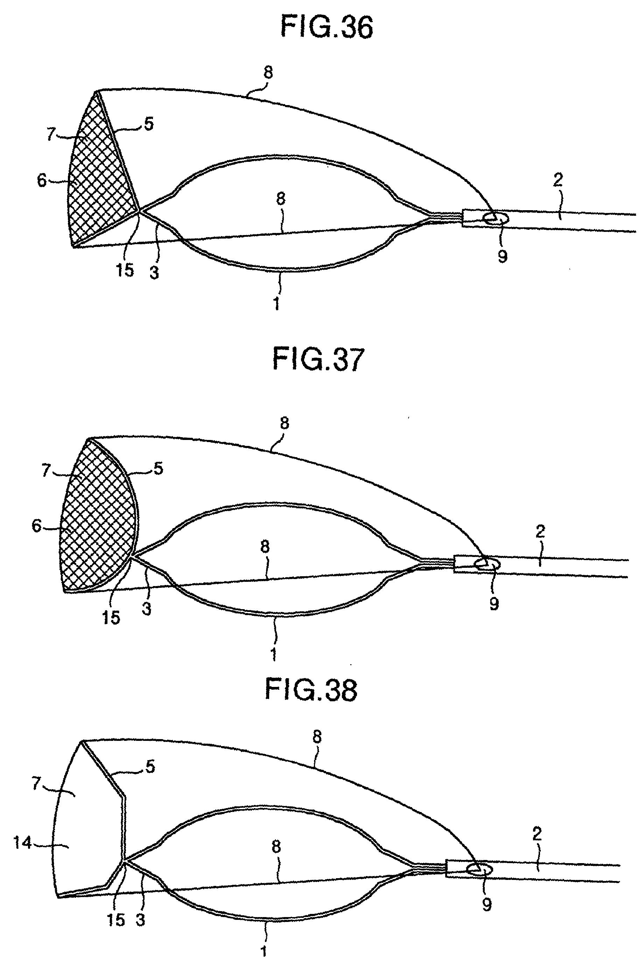

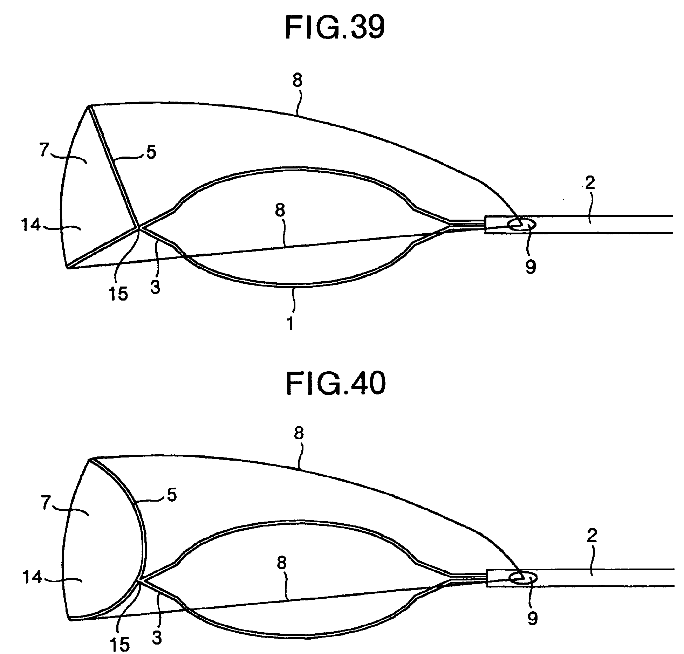

A shape of the collecting device 7 is not limited, in accordance with the present embodiment, it is sufficient to consider that the front end turning point 15 is provided so that the collecting device 7 forms a bending shape covering the diseased portion 10 from the above at a time of fixing the diseased portion 10 as mentioned above and this can collect an expected size of the diseased portion 10. As embodiments of the shape of the collecting device 7, the following structures are listed up. Fig. 36 shows a structure having the collecting device 7 formed in a triangular shape as a whole constituted by the net frame 5 having no bent portion, and Fig. 37 shows a structure in which the net frame 2 is bent in a circular arc shape and the collecting device 7 formed in a meniscus shape as a whole is provided. Further, in place of the net 6 mentioned above, a thin sheet 14 may be employed. Fig. 38 shows a structure having the collecting device 7 formed in a pentagonal shape as a whole in the same manner as that shown in Fig. 28, Fig. 39 shows a structure having the collecting device 7 formed in a triangular shape as a whole in the same manner as that shown in Fig. 36, and Fig. 40 shows a structure having the collecting device 7 formed in a meniscus shape as a whole in the same manner as that shown in Fig. 37.

The collecting device 7 requires a size capable of covering the expected diseased portion 10 so as to fix, that is, if the expected size of the diseased portion 10 is 5 to 15 mm, it is recommended that a width of the collecting device 7 is 10 to 20 mm and a length thereof is about 12 to 25 mm, however, this is not limited.

Further, it is preferable that a position of the opening portion 9 is set so that a distance between the front end turning point 15 and the opening portion 9 becomes substantially equal to a distance between the front end turning point 15 and the connecting portion of the collecting device 7 to the collecting device operating fiber 8 at a time of fixing the diseased portion 10 as shown in Fig. 34, and it is preferable to arrange within a range between 2 and 20 mm from the leading end of the sheath tube 2 and more desirably within a range between 2 and 15 mm.

Due to the opening portion 9, since an intended motion of the collecting device 7 as shown in Fig. 34 can be achieved, and a distance of the collecting device operating fiber 8 from the leading end portion of the sheath tube 2 generating a high heat at a time of cauterizing shown in Fig. 35 can be secured so that the collecting device operating fiber 8 is not broken due to the heat, there can be obtained an effect that no specific heat resisting property is not required for the collecting device operating fiber 8.

### "Embodiments of each of constituting elements"

It is preferable that the snare wire is made of a conductive material capable of applying a high-frequency electric current, a diameter at a time of opening the loop is the same size as the generally used snare loop, and the snare wire is constituted by a single track stainless wire or a double track stainless wire having an outer diameter of 0.2 to 1.0 mm for moving in and out within the sheath tube 2, however, as far as it has a restoring property capable of keeping a loop shape of the snare, an operability of the wire in accordance with a remote control, a certain level of rigidity, a rust proofing property against contact with a body fluid within the body cavity and a hardness against giving influence to the inner portion of the body, any material may be employed.

It is necessary that the net frame 5 is made of a material having an insulating property, a high maintaining property of a set shape, and a suitable rigidity and flexibility capable of bending at the turning point 4 or the front end turning point 15 so as to deform the collecting portion 7. It is not said to limit, however, a polyvinyl chloride resin, a fluorine resin, a polyethylene resin, a polypropylene resin, a nylon resin and the like are preferable. Further, the snare wire may be extended and may be used by insulating coating. Further, when the collecting device 7 is received in the inner hole of the sheath tube 2 for a long time, there is a risk that the shape is not sufficiently restored at a time of being taken out from the sheath tube 2, so that it is preferable that a nickel titanium having a shape memory function is used by insulating treating a surface thereof. An outer diameter of the net frame 5 is preferably set to 0.2 to 1.0 mm for the purpose of moving in and out within the sheath tube 2.

The thread forming the net 6 fixed to the net frame 5 is not directly exposed to the heat at a time of cauterizing by the high-frequency, however, it is preferable to employ a heat resisting resin having a melting point of 160°C or more such as a polyester resin fiber, a nylon resin fiber, an aromatic polyamide fiber and the like. The diameter of the thread of the net is preferably set to about 10 to 40 micron-with considering taking in and out within the sheath tube 2. A mesh of the net may be set in correspondence to a minimum size of the diseased portion 10 in the present embodiment, and if it is set to 5 mm, it is preferable that the width of the mesh is set to be equal to or less than 3 mm. Further, in general, the net 6 may be formed as a fiber shape netted so as to having the binding points, may be formed by crossing the fiber and welding the intersecting points in accordance with the high frequency or the like or may be formed by resecting a sheet-like member in accordance with a laser beam or punching by a pressing operation so as to form windows, however, no limitation exists as far as the required matters mentioned above are satisfied.

Further, at a time of using the sheet 14 for the collecting device 7, the material of the sheet 14 is not directly exposed to the heat at a time of cauterizing in accordance with the high frequency in the same manner as the net 6, however, it is preferable to employ a heat resisting resin having a melting point of 160°C or more such as a polyester resin fiber, a nylon resin fiber, an aromatic polyamide fiber and the like, and a thickness of the sheet is preferably set to about 10 to 30 micron with considering taking in and out within the sheath tube 2.

There is no risk that the collecting device operating fiber 8 is directly exposed to the heat at a time of cauterizing in accordance with the high frequency, however, it is preferable to employ a heat resisting resin having a melting point of 160°C or more such as a polyester resin fiber, a nylon resin fiber, an aromatic polyamide fiber and the like. However, as shown in Fig. 27, in the case that the collecting device operating fiber 8 is introduced outward from the inner hole of the sheath tube 2 at the leading end of the sheath tube 2, since there is a high possibility that the collecting device operating fiber 8 is exposed to the heat, an aromatic polyester fiber, an aramid fiber or the like having a high heat resisting property is preferable. An outer diameter thereof is preferably set to about 100 to 200 micron with considering taking in and out within the sheath tube 2 and a tensile strength sufficient to deform the net frame 5 or the collecting device 7.

In accordance with the embodiment of the snare with the collecting device of the present invention, the snare portion has a front face and a back face, and it is necessary for the user to know the front and back faces under an image of the endoscope. Particularly, in the narrow tube hole of the alimentary canal, there is a case that the front and back faces are known on the basis of a positional relation between the collecting device operating fiber 8 positioned close to the endoscope lens 13 and the sheath tube 2, and it is necessary that the collecting device operating fiber 8 has striking colors within the body of the patient corresponding to a used environment. In accordance with the present embodiment, no limitation is particularly given, however, a recommended color for the collecting device operating fiber 8 is a red color, a blue color, a green color, a white color or a yellow color. Further, by combining two to five colors among them so as to form a stripe shape, it becomes easy to differentiate on a visual field from the snare wire of the snare loop 1, so that it is possible to further expect the effect.

Since the sheath tube 2 has an improved slippage with the snare portion and the inner wall of the forceps hole 12 of the endoscope 11, and the snare portion is operated by rotating the sheath tube 2 of changing the direction or on the basis of the remote control, a polyvinyl chloride resin having a rigidity, a fluorine resin, a polyethylene resin and a polypropylene resin are preferable, however, the other resins with which a metal coil, a metal mesh or the like is composed for keeping a rigidity may be employed, and the kind of the resin is not particularly limited. The outer diameter in accordance with the present embodiment is set to about 2 to 2.5 mm with considering the inner diameter of the forceps hole 12 of the endoscope 11.

### "Embodiment 1 of handle portion"

The handle portion mentioned above requires a function of moving forward and backward the snare loop 1 and the collecting device operating fiber 8 in a remote control manner. The means is not limited, however, embodiments will be shown below.

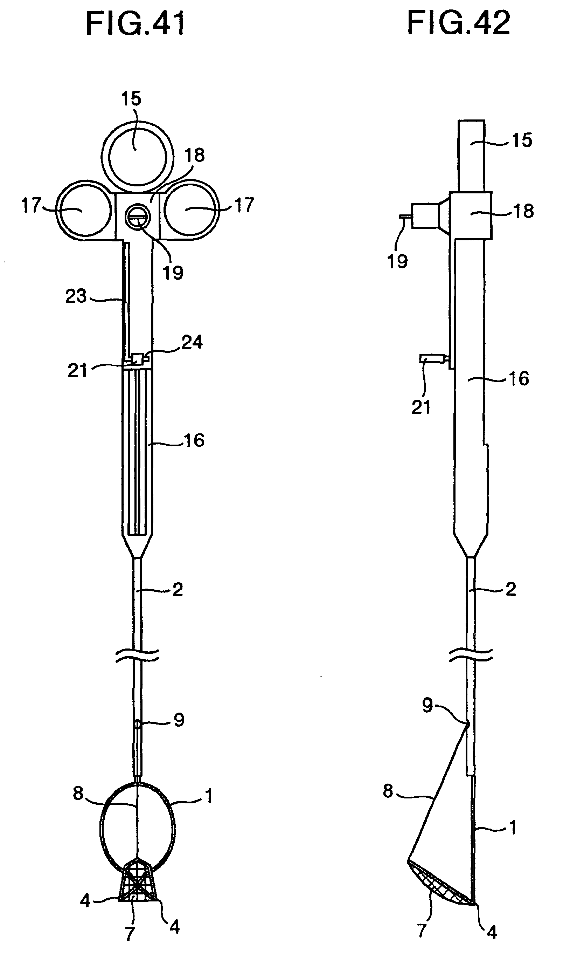

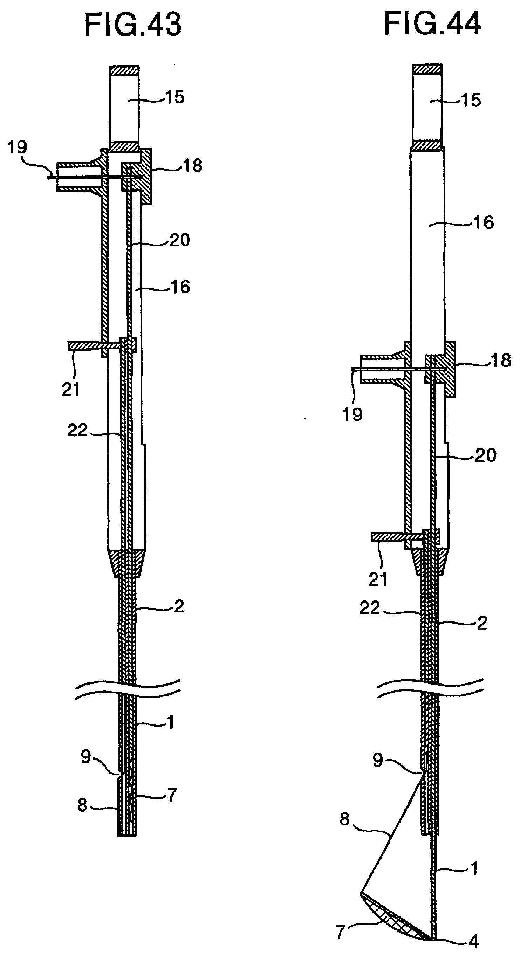

Fig. 41 is a top elevational view of an embodiment of the handle portion, Fig. 42 is a side elevational view thereof, and Figs. 43 to 46 are schematic view of an inner portion showing a procedure of operation. At first, the handle portion has a handle main body 16 integrally formed with a rearward finger engaging portion 15, and a rear end of the sheath tube 2 is connected to a forward portion of the handle portion. Further, a slide portion 18 capable of sliding in a longitudinal direction of the handle main body 16 and integrally formed with a forward finger engaging portion 17 is provided in the handle main body 16. As shown in Fig. 43, a conductive electrode 19 is provided in the slide portion 18, and a rear end of a conductive pipe-shaped or rod-shaped snare portion driving rod 20 arranged so as to be received within the handle main body 16 is connected to the electrode 19. A front end of the snare portion operating rod 20 is inserted into the inner hole of the sheath tube 2, and is connected to a conductive snare driving wire connected to the snare loop 1 therewithin. At this time, the electrode 19, the snare portion operating rod 20, the snare driving wire and the snare loop 1 are electrically conducted.

A collecting device operating lever 21 fixed to the slide portion 18 and capable of sliding in a longitudinal direction within the slide portion 18 within a fixed distance is provided in the slide portion 18. In accordance with the present embodiment, the collecting device operating lever 21 is connected to the snare portion operating rod mentioned above so as to freely slide in the longitudinal direction, thereby making it stable to fix the collecting device operating lever 21. A collecting device operating rod 22 is connected and fixed to the collecting device operating lever 21, and a front end of the collecting device operating rod 22 is inserted into the inner hole of the sheath tube 2 and is connected to the collecting device driving wire connected to the collecting device operating fiber 8 therewithin.

A description will be given of a method of operating the handle portion in accordance with the present embodiment. At first, Fig. 43 shows a state that the collecting device 7 and the snare portion are received within the sheath tube 2 as shown in Figs. 2, 15 and 29. At this time, the slide portion becomes in a state of being drawn to the rear end on the handle main body 16, and the collecting device operating lever 21 becomes in a state of being moved to the front end on the slide portion 18. At this time, the collecting device operating lever 21 is moved along a route restricted by a groove-like slide groove 23 provided in the slide portion 18, and the collecting device operating lever 21 is engaged with a slide lock groove 24 provided at a front end of the slide groove 23 so as to temporarily fix the slide portion 18.

Fig. 44 shows a state that the collecting device 7 and the snare portion received in the sheath tube 2 are out of the sheath tube 2, as shown in Figs. 3, 16 and 30. At this time, while keeping the state that the collecting device operating lever 21 is temporarily fixed to the slide portion 18 by the slide lock groove 24, the slide portion 18 becomes in a state of being pressed out to the front end on the handle main body 16. On the way to moving to the state, the slide portion 18 is forward pressed out, whereby the snare portion operating rod 20 is pressed and the snare loop 1 is pressed out. At the same time, the collecting device operating lever 21 temporarily fixed to the slide portion 18 is also pressed, the collecting device operating rod 22 is pressed and the collecting device operating fiber 8 is also pressed out by the opening portion 9.

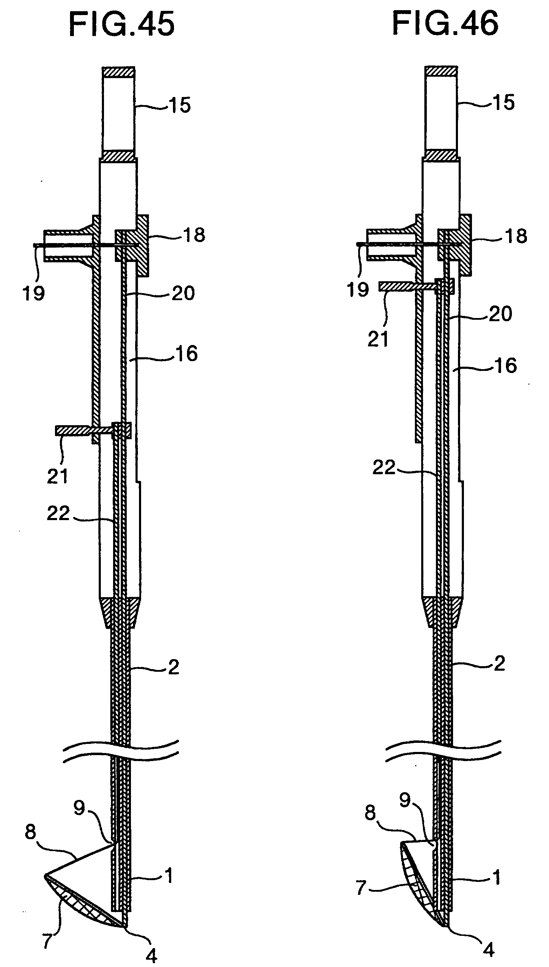

Fig. 45 shows a state of strangulating the diseased portion 10, as shown in Figs. 6, 19 and 33. At this time, while keeping the state that the collecting device operating lever 21 is temporarily fixed to the slide portion 18 by the slide lock groove 24, the slide portion 18 becomes in a state of being drawn to the rear end on the handle main body 16. On the way to moving to the state, the slide portion 18 is rearward drawn, whereby the snare portion operating rod 20 and the snare loop 1 are drawn and are received in the inner hole of the sheath tube 2. At the same time, the collecting device operating lever 21 temporarily fixed to the slide portion 18 is also drawn, the collecting device operating rod 22 and the collecting device operating fiber 8 are also drawn so as to be drawn in from the opening portion 9.

Fig. 46 shows a state of fixing the diseased portion 10 by the collecting device 7, as shown in Figs. 7, 20 and 34. At this time, the collecting device operating lever 21 is cancelled from the fixing state to the slide lock groove 24 and is moved rearward along the slide groove 23 on the slide portion 18. On the way to moving to the state, the collecting device operating lever 21 is drawn, whereby the collecting device operating rod 22 and the collecting device operating fiber 8 are also drawn and the collecting device 7 executes a tilting operation around the turning point 4.

Generally, the handle portion in accordance with the present embodiment is operated for a user by inserting a first finger and a second finger to the forward finger engaging portion 17 and inserting a thumb to the rearward finger engaging portion 15 by one hand, however, a distance at which the slide portion 18 moves along the handle main body 16 requires a length capable of receiving at least the snare loop 1 and the collecting device 7 with taking the size of the hand of the operator into consideration, and accordingly, it is necessary to take a distance obtained by extending both elements on a straight line into consideration. In accordance with the present embodiment, it is recommended that this part is 60 to 100 mm, desirably 60 to 80 mm.

A distance at which the collecting device operating lever 21 moves along the slide portion 18 requires a moving amount of the collecting device operating fiber 2 necessary for the collecting device 7 to tilt down in such a manner as to fix the diseased portion 10 as shown in Figs. 8, 20 and 34 from the state of strangulating the diseased portion 10 as shown in Figs. 6, 19 and 33, and in the present embodiment, a distance 20 to 50 mm is recommended.

The handle main body 16, the slide portion 18 and'the collecting device operating lever 21 requires a rigidity sufficiently standing against the driving operation of the snare operating rod 20 and the collecting device operating rod 22 and an insulating property capable of insulating the high-frequency electric current, and taking a productivity into consideration, an injection molded product of a polycarbonate resin, a hard vinyl chloride and a polyethylene resin is recommended, however, it is not limited.

The snare operating rod 20 requires a rigidity capable of standing against the load at a time taking the snare loop 1 and the collecting device 7 in and out the inner hole of the sheath tube 2 and a conductivity capable of transmitting the high-frequency electric current. In this case, since there is a possibility that the user is in contact with the surface, it is preferable to apply an insulating treatment to the surface. A stainless steel pipe or rod is preferably employed for a material thereof.

It is necessary to form the electrode so as to correspond to a standard of a connecting code of a high-frequency cauterizing apparatus supplying a high-frequency electric current. In accordance with the present embodiment, a press punched product of a stainless steel is used, however, a resected product or the like may be employed, and no limitation exists as far as a conductivity can be obtained.

### "Embodiment 2 of handle portion"

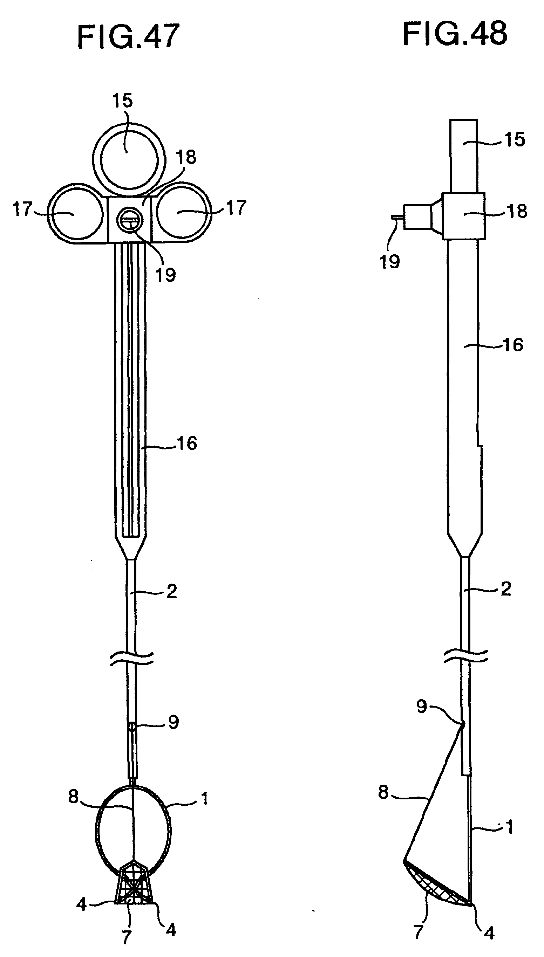

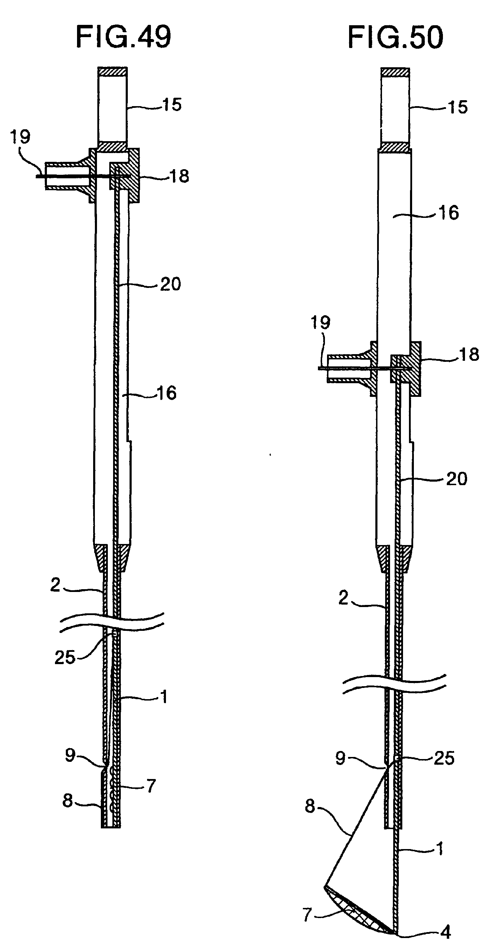

Fig. 47 is a top elevational view of another embodiment of the handle portion, Fig. 48 is a side elevational view thereof, and Figs. 49 to 52 are schematic view of an inner portion showing a procedure of operation. At first, the handle portion has a handle main body 16 integrally formed with a rearward finger engaging portion 15, and a rear end of the sheath tube 2 is connected to a forward portion of the handle portion. Further, a slide portion 18 capable of sliding in a longitudinal direction of the handle main body 16 and integrally formed with a forward finger engaging portion 17 is provided in the handle main body 16. As shown in Fig. 49, a conductive electrode 19 is connected to the slide portion 18, and a rear end of a conductive pipe-shaped snare portion driving rod 20 arranged so as to be received within the handle main body 16 is connected to the electrode 19. A front end of the snare portion operating rod 20 is inserted into the inner hole of the sheath tube 2, and is connected to a conductive snare driving wire connected to the snare loop 1 therewithin. At this time, the electrode 19, the snare portion operating rod 20, the snare driving wire and the snare loop 1 are electrically conducted.

Further, a collecting device operating fiber connecting portion 25 is connected and fixed to the snare driving wire, and another end of the collecting device operating fiber 8 connected to the collecting device 7 is connected to the collecting device operating fiber connecting portion 25.

A description will be given of a method of operating the handle portion in accordance with the present embodiment. At first, Fig. 49 shows a state that the collecting device 7 and the snare portion are received within the sheath tube 2 as shown in Figs. 2, 15 and 29. At this time, the slide portion becomes in a state of being drawn to the rear end on the handle main body 16.

Fig. 50 shows a state that the collecting device 7 and the snare portion received in the sheath tube 2 are out of the sheath tube 2, as shown in Figs. 3, 16 and 30. At this time, the slide portion 18 becomes in a state of being pressed out to the front end on the handle main body 16. On the way to moving to the state, the slide portion 18 is forward pressed out, whereby the snare portion operating rod 20 and the snare driving wire are pressed and the snare loop 1 is pressed out. At the same time, the collecting device operating fiber 8 connected to the snare driving wire by the collecting device operating fiber connecting portion 25 is also pressed out by the opening portion 9.

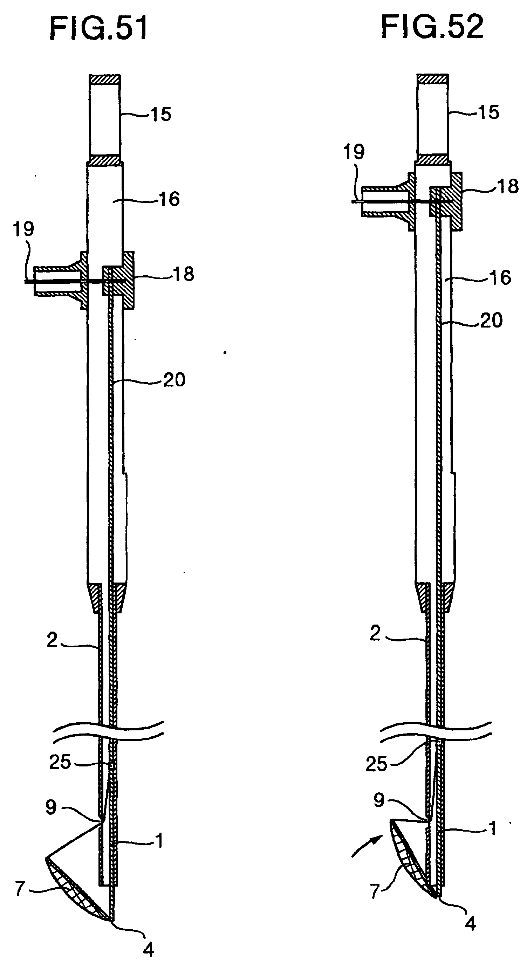

Fig. 51 shows a state of strangulating the diseased portion 10. At this time, the slide portion 18 becomes in a state of being drawn to the rear end on the handle main body 16. At this time, the slide portion 18 is rearward drawn, whereby the snare portion operating rod 20, the snare driving wire and the snare loop 1 are drawn and are received in the inner hole of the sheath tube 2. At the same time, the collecting device operating fiber 8 connected and fixed to the snare driving wire by the collecting device operating fiber connecting portion 25 is also drawn in from the opening portion 9, and the collecting device 7 starts a tilt-down operation toward the center of the turning point 4. When strangulating further, the collecting device 7 fully tilts down as shown in Fig. 52 showing the same state in Figs. 7, 20 and 34, thereby becoming the state of fixing the diseased portion 10 by the collecting device 7.

Generally, the handle portion in accordance with the present embodiment is operated for a user by inserting a first finger and a second finger to the forward finger engaging portion 17 and inserting a thumb to the rearward finger engaging portion 15 by one hand, however, a distance at which the slide portion 18 moves along the handle main body 16 requires a length capable of receiving at least the snare loop 1 and the collecting device 7 with taking the size of the hand of the operator into consideration, and accordingly, it is necessary to take a distance obtained by extending both elements on a straight line into consideration. In accordance with the present embodiment, it is recommended that this part is 60 to 100 mm, desirably 60 to 80 mm.

The handle main body 16 and the slide portion 18 requires a rigidity sufficiently standing against the driving operation of the snare operating rod 20 and an insulating property capable of insulating the high-frequency electric current, and taking a productivity into consideration, an injection molded product of a polycarbonate resin, a hard vinyl chloride and a polyethylene resin is recommended, however, it is not limited.

It is necessary to form the electrode so as to correspond to a standard of a connecting code of a high-frequency cauterizing apparatus supplying a high-frequency electric current. In accordance with the present embodiment, a press punched product of a stainless steel is used, however, a resected product or the like may be employed, and no limitation exists as far as a conductivity can be obtained.

### "Embodiment 3 of handle"

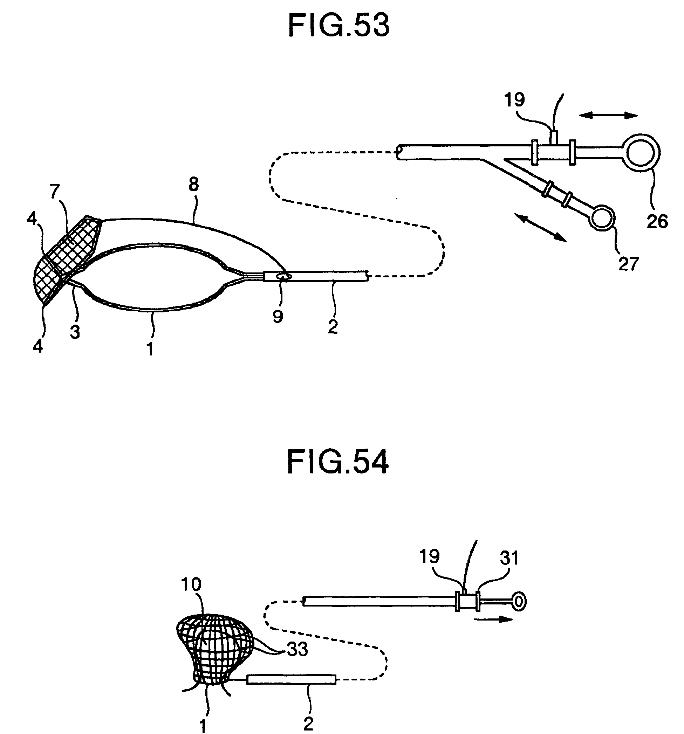

Further, the handle portion may be structured such as to independently perform an operation of the snare loop 1 and an operation of the collecting device 7, as shown in Fig. 53. A snare portion operating handle 26 is connected to the snare loop 1 at a front end via the snare portion operating wire and can move the snare loop 1 forward and backward in correspondence to the forward and backward movement of the snare portion operating handle 26, and the collecting device operating handle 27 is connected to the collecting device operating fiber 8 at the front end via the collecting device operating wire and can move the collecting device operating fiber 8 forward and backward in correspondence to the forward and backward movement of the collecting device operating handle 27, thereby controlling a tilting way of the collecting device 7.

### INDUSTRIAL APPLICABILITY

Since it is possible to collect the diseased portion at the same time of resecting in the conventional endoscopic ablative operation of the polyp or the like, by using the snare with the collecting device in accordance with the present invention, there is no case of losing sight of the diseased portion during the operation, thereby incapable of collecting, the cauterizing capacity of the snare itself is not prevented, the net is not broken by being melted due to the heat of cauterization, and it is possible to easily and securely resect and retrieve the diseased portion under a good endoscopic sight.

## Claims

1. A snare with a collecting device comprising:
a snare wire made of a conductive wire and forming a loop portion at a front end;
said snare wire being connected to a handle portion at a rear end through a sheath tube;
the snare loop being moved forward and backward by operating the handle portion; and
a high-frequency snare received in the sheath tube and resecting a diseased portion such as a polyp or the like generated in a tissue within a body cavity in an endoscopic manner,
wherein the collecting device is provided at a leading end of the loop portion of the snare wire, and one or a plurality of collecting device operating fibers are connected to the collecting device.

2. A snare with a collecting device as claimed in claim 1, wherein the collecting device is constituted by a net, or a sheet or a combination thereof.

3. A snare with a collecting device as claimed in claim 1, wherein the collecting device has a turning point and has a function of bending at the turning point.

4. A snare with a collecting device as claimed in claim 3, wherein the collecting device has a turning point and has a function that the collecting device bends in a vertical direction at the turning point.

5. A snare with a collecting device as claimed in claim 1, wherein the collecting device has a turning point and has a function that the collecting device bends in a vertical direction and a longitudinal direction at the turning point.

6. A snare with a collecting device as claimed in claim 1, wherein the collecting device operating fiber is red, blue, green, white or yellow.

7. A snare with a collecting device as claimed in claim 1, wherein the collecting device operating fiber is red, blue, green, white or yellow and two colors or a plurality of colors among them form a stripe-like arranged color.

8. A snare with a collecting device as claimed in claim 1, wherein the collecting device operating fiber is introduced to an outer portion from an inner hole of the sheath tube via an opening hole for communicating an outer surface of the sheath tube at the rear of the front end of the sheath tube with the inner hole.

9. A snare with a collecting device as claimed in claim 1, wherein a collecting device operating rod provided in the handle portion is connected to a snare operating rod provided in the handle portion in the same manner.

10. A snare with a collecting device as claimed in claim 1, wherein a collecting device operating rod provided in the handle portion is connected to a snare operating rod provided in the handle portion in the same manner and a relative position between both of the operating rods is capable of being changed within a fixed distance.
